# EUROPEAN PATENT APPLICATION

(11) **EP 4 197 592 A1**
(43) Date of publication of application: **21.06.2023**
(21) Application number: 21857563.7
(22) Date of filing: 12.08.2021
(51) Int. Cl.: A61N 5/10

(54) **RADIOACTIVE RAY RADIATION SYSTEM AND CONTROL METHOD THEREFOR**

(30) Priority: 15.08.2020 CN 202010821753
(71) Applicant: Neuboron Therapy System Ltd., Xiamen, Fujian 361026 (CN)
(72) Inventor: CHEN, Jiang, Nanjing, Jiangsu 211112 (CN)
(74) Representative: Gunzelmann, Rainer
(86) International application number: PCT/CN2021/112197
(87) International publication number: WO 2022/037468

(57) **Abstract**

Provided are a radioactive ray radiation system and a control method therefor. The radioactive ray radiation system comprises a beam radiation apparatus, a treatment planning module, a control module, a preparation room and a radiation room, wherein a first stereoscopic vision apparatus and a second stereoscopic vision apparatus are respectively arranged in the preparation room and the radiation room. Simulated positioning is performed on a radiated subject in the preparation room according to the location of a radiated part determined in a treatment plan, and a first image of the radiated part that is collected by the first stereoscopic vision apparatus is compared with the treatment plan so as to determine a simulated positioning pose; and radiation positioning is performed on the radiated subject in the radiation room according to the determined simulated positioning pose, and a second image of the radiated part that is collected by the second stereoscopic vision apparatus is compared with the treatment plan so as to control the beam radiation apparatus to start performing radiation therapy on the radiated subject. By means of providing stereoscopic vision apparatuses, it is ensured that radiation therapy is performed on a radiated subject according to a treatment plan, thereby achieving an expected therapeutic effect, and also reducing radiation injuries for normal tissue.

## Description

### TECHNICAL FIELD

An aspect of the invention relates to a radioactive ray irradiation system; and another aspect of the invention relates to a control method for a radioactive ray irradiation system.

### BACKGROUND

With the development of atomics, radioactive ray therapy, such as cobalt sixty, a linear accelerator, an electron beam, or the like, has become one of the major means to treat cancers. However, traditional photon or electron therapy is restricted by physical conditions of radioactive rays themselves, and thus will also harm a large number of normal tissues on a beam path while killing tumor cells. Furthermore, owing to different levels of sensitivity of tumor cells to radioactive rays, traditional radiotherapy usually has poor treatment effect on malignant tumors (for example, glioblastoma multiforme and melanoma) with radio resistance.

In order to reduce radiation injury to normal tissues around tumors, a target therapy concept in chemotherapy is applied to radioactive ray therapy. With respect to tumor cells with high radio resistance, irradiation sources with high relative biological effectiveness (RBE), such as proton therapy, heavy particle therapy, neutron capture therapy, or the like, are also developed actively now Here neutron capture therapy combines the abovementioned two concepts, for example, boron neutron capture therapy (BNCT), provides a better cancer treatment choice than traditional radioactive rays, by specific aggregation of boron-containing drugs in tumor cells in combination with precise neutron beam regulation and control.

In the radioactive ray therapy, precise treatment is implemented by aligning the beam to tumor cells in an irradiated body on a treatment table, and radiation injuries to normal tissues around tumor cells of the irradiated body are reduced to the greatest extent. In a clinical treatment process, a patient's irradiated body is required to be accurately positioned according to a treatment solution determined according to a treatment plan strictly, and when the position has a too large error, a central position of a target area of a focus of infection is changed, so that a desired treatment effect cannot be achieved. Furthermore, such as for BNCT, one irradiation takes approximately half an hour, and unconscious movement of the patient's irradiated body in this process may also result in change of the central position of the target area of the focus of infection.

Therefore, it is necessary to propose a new technical solution to solve the above-mentioned problems.

### SUMMARY

In order to solve the above-mentioned problems, one aspect of the invention provides a radioactive ray irradiation system, including a beam irradiation device, a treatment plan module, a control module, a preparation room and an irradiation room. The beam irradiation device generates a treatment beam and irradiates the treatment beam to an irradiated body to form an irradiated site. The treatment plan module performs dose simulation and calculation according to parameters of the treatment beam and medical image data of the irradiated site and generates a treatment plan which determines a position of the irradiated site relative to the beam irradiation device during irradiation treatment. The control module controls irradiation of the beam irradiation device according to the treatment plan. Simulated positioning of the irradiated body is performed in the preparation room according to the position of the irradiated site determined by the treatment plan, and a first stereoscopic vision device is arranged in the preparation room to collect a first image of the irradiated site, and the control module compares the first image with the position of the irradiated site determined by the treatment plan, to ensure that the comparison result is in an allowable difference range and determine a simulated positioning pose of the irradiated body. Irradiation position of the irradiated body is performed in the irradiation room according to the determined simulated positioning pose, and a second stereoscopic vision device is arranged in the irradiation room to collect a second image of the irradiated site, the control module compares the second image with the first image of the irradiated site corresponding to the determined simulated positioning pose or the position of the irradiated site determined by the treatment plan, to ensure that the comparison result is in an allowable difference range, and control the beam irradiation device to start irradiation treatment of the irradiated body. The simulated positioning in the preparation room saves operation time of positioning the irradiated body before irradiation treatment in the irradiation room, and irradiation treatment of another irradiated body may be performed while performing preparation operations, increasing utilization rate of the device; and the stereoscopic vision devices are provided to collect images of the irradiated body before simulated positioning and irradiation treatment and compare the images of the irradiated body with the treatment plan, to ensure irradiation treatment of the irradiated body according to the treatment plan, and achieve an expected treatment effect, while radiation injuries to normal tissues are reduced.

Preferably, the beam irradiation device may include a beam outlet at least partially arranged in the irradiation room, the preparation room is provided with a simulated beam outlet which is the same as the beam outlet, a positional relationship of the simulated beam outlet and the first stereoscopic vision device is the same as that of the beam outlet and the second stereoscopic vision device, the preparation room and the irradiation room define the same irradiation coordinate system therein, and the control module is capable of converting each of the first image and the second image into a coordinate matrix of the irradiated site in the irradiation coordinate system.

Further, the treatment plan module may perform dose simulation and calculation by using a Monte Carlo simulation program, the treatment plan module converts the medical image data of the irradiated site into a voxel prosthesis tissue model required by the Monte Carlo simulation program, the medical image data of the irradiated site includes a coordinate matrix of the irradiated site in a medical image coordinate system, and the treatment plan module or the control module is capable of acquiring a coordinate conversion matrix of the medical image coordinate system and the irradiation coordinate system.

Further, the irradiated site may be provided with a feature pattern made of a material which is capable of being developed by a medical image and being recognized by the first stereoscopic vision device and the second stereoscopic vision device, each of the first stereoscopic vision device and the second stereoscopic vision device collects an image of the irradiated site by collecting an image of the feature pattern, and the control module converts the image of the feature pattern into the coordinate matrix of the irradiated site in the irradiation coordinate system according to a position of the feature pattern in a medical image of the irradiated site. The feature pattern is provided, so that data collected by the stereoscopic vision devices is faster and more accurate.

Further, each of the preparation room and the irradiation room may be provided with the same laser positioning device having the same positional relationship, and the treatment plan module is capable of simulating a position of a laser generated by the laser positioning device hitting the irradiated site. An operator marks on the irradiated body according to the position, and adjusts position and posture of simulated positioning of the irradiated body in the preparation room and position and posture of irradiation positioning of the irradiated body in the irradiation room according to the mark, so that the position of the laser generated by the laser positioning device hitting the irradiated body is consistent with that of the mark, and the laser positioning device is provided, so that manually positioning is more convenient and faster for the operator. Furthermore, the laser generated by the laser positioning device may determine a position consistent with central axes of the beam outlet and the simulated beam outlet. The position of the laser generated by the laser positioning device hitting the irradiated body represents a position where a central axis of a beam emitted from the beam outlet is incident on the irradiated body, and a beam central axis simulated according to the treatment plan is marked on the irradiated body at an incident point of the voxel prosthesis tissue model, so that incident positions of beams determined during the simulated positioning and the irradiation treatment are more accurate.

Further preferably, the radioactive ray irradiation system may further include a treatment table and a treatment table positioning device arranged in the irradiation room, the irradiated body is subject to irradiation treatment on the treatment table, and the control module controls movement of the treatment table through the treatment table positioning device.

Further preferably, the second stereoscopic vision device may collect a third image of the irradiated site in real time during irradiation treatment, and the control module compares the third image with a corresponding second image of the irradiated site when the irradiation treatment is started, or the first image of the irradiated site corresponding to the determined simulated positioning pose or the position of the irradiated site determined by the treatment plan, to ensure that the comparison result is in an allowable difference range, and control the beam irradiation device to continuously perform the irradiation treatment of the irradiated body. The stereoscopic vision device is provided to collect the image of the irradiated body in real time during the irradiation treatment and compare the image of the irradiated body with the treatment plan, to ensure that irradiation treatment of the irradiated body is always performed according to the treatment plan during the irradiation treatment, and a better treatment effect is obtained.

Further preferably, the radioactive ray irradiation system may be a neutron capture therapy system, the beam irradiation device may include a neutron generation device, a beam shaping body and a treatment table. The neutron generation device includes an accelerator and a target, the accelerator accelerates charged particles to generate a charged particle line which acts with the target to generate a neutron line. The beam shaping body is capable of adjusting the neutron line generated by the neutron generation device to a preset beam quality. On the treatment table, the irradiated body is irradiated by the neutron line generated by the neutron generation device through the beam shaping body.

Another aspect of the invention provides a control method for a radioactive ray irradiation system, the radioactive ray irradiation system includes a beam irradiation device, a treatment plan module, a control module, a preparation room and an irradiation room. The beam irradiation device generates a treatment beam and irradiates the treatment beam to an irradiated body to form an irradiated site. In the preparation room and the irradiation room, a first stereoscopic vision device and a second stereoscopic vision device are arranged respectively. The control method includes the following operations. The treatment plan module performs dose simulation and calculation according to parameters of the treatment beam generated by the beam irradiation device and medical image data of the irradiated site, and generates a treatment plan which determines a position of the irradiated site relative to the beam irradiation device during irradiation treatment and a corresponding irradiation time. The control module retrieves a current treatment plan corresponding to the irradiated body from the treatment plan module. Simulated positioning of the irradiated body is performed in the preparation room according to the position of the irradiated site determined by the treatment plan. The first stereoscopic vision device collects a first image of the irradiated site, and the control module compares the first image with the position of the irradiated site determined by the treatment plan, to ensure that the comparison result is in an allowable difference range and determine a simulated positioning pose of the irradiated body. Irradiation positioning of the irradiated body is performed in the irradiation room according to the determined simulated positioning pose. The second stereoscopic vision device collects a second image of the irradiated site, and the control module compares the second image with the first image of the irradiated site corresponding to the determined simulated positioning pose or the position of the irradiated site determined by the treatment plan, to ensure that the comparison result is in an allowable difference range, and control the beam irradiation device to start irradiation treatment of the irradiated body. In response to reaching irradiation time determined by the treatment plan, the control module controls the beam irradiation device to stop irradiation of the irradiated body.

Preferably, the control method may further include the following operations. After the beam irradiation device starts the irradiation treatment of the irradiated body, the second stereoscopic vision device collects a third image of the irradiated site in real time during the irradiation treatment, and the control module compares the third image with a corresponding second image of the irradiated site when the irradiation treatment is started, or the first image of the irradiated site corresponding to the determined simulated positioning pose or the position of the irradiated site determined by the treatment plan, to ensure that the comparison result is in an allowable difference range, and control the beam irradiation device to continuously perform the irradiation treatment of the irradiated body.

Further preferably, the control method may further the following operations. The same irradiation coordinate system is defined in the preparation room and the irradiation room. The treatment plan module converts the medical image data of the irradiated site into a voxel prosthesis tissue model, and the treatment plan module converts the position of the irradiated site determined by the treatment plan into a coordinate matrix of the voxel prosthesis tissue model of the irradiated site in the irradiation coordinate system.

Further, the control method may further include the following operations. The control module converts the first image and the second image into the irradiation coordinate system, for comparison.

Further, the control method may further include the following operations. A feature pattern is provided on the irradiated site, and a position of the feature pattern in a medical image of the irradiated site is collected. Furthermore, the control method may further include the following operations. The first stereoscopic vision device and the second stereoscopic vision device collect images of the feature pattern, and the first stereoscopic vision device and the second stereoscopic vision device transmit the images to the control module. The control module converts the images into coordinate matrices of the irradiated site in the irradiation coordinate system through the position of the feature pattern in the medical image of the irradiated site, for comparison.

According to the radioactive ray irradiation system and the control method therefor, the stereoscopic vision devices are provided to collect images of the irradiated body before simulated positioning and irradiation treatment and compare the images of the irradiated body with the treatment plan, to ensure irradiation treatment of the irradiated body according to the treatment plan, and achieve an expected treatment effect, while radiation injuries to normal tissues are reduced.

### BRIEF DESCRIPTION OF THE DRAWINGS

FIG. 1 is a schematic module diagram of a BNCT system according to an embodiment of the invention.
FIG. 2 is a schematic structural diagram of a BNCT system according to an embodiment of the invention.
FIG. 3 is a schematic diagram of a BNCT system when an irradiated body is positioned according to an embodiment of the invention.
FIG. 4 is a flowchart of an operation process of a BNCT system according to an embodiment of the invention.
FIG. 5 is a flowchart of a treatment plan module of a BNCT system generating a treatment plan according to an embodiment of the invention.

### DETAILED DESCRIPTION

Embodiments of the invention will be further described in detail below with reference to the drawings, to enable those skilled in the art to implement the embodiments with reference to texts of the description.

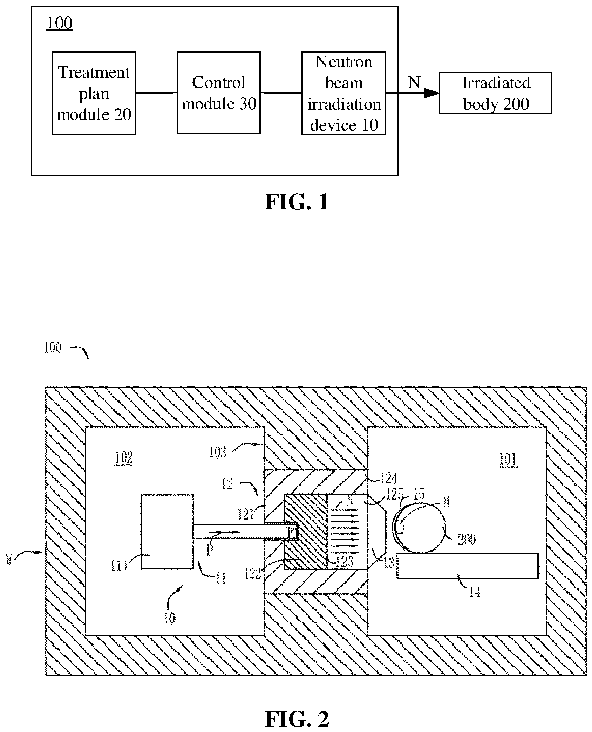

As shown in FIG. 1, the radioactive ray irradiation system in this embodiment is a BNCT system 100, and includes a neutron beam irradiation device 10, a treatment plan module 20 and a control module 30. The neutron beam irradiation device 10 generates a treatment neutron beam N and irradiates the treatment neutron beam N to an irradiated body 200 to form an irradiated site. The treatment plan module 20 performs dose simulation and calculation according to parameters of the treatment neutron beam N generated by the neutron beam irradiation device 10 and medical image data of the irradiated site and generates a treatment plan which determines a position of the irradiated site relative to the neutron beam irradiation device 10 during irradiation treatment and a corresponding irradiation time. After the irradiated body 200 is positioned according to the position determined by the treatment plan, treatment may be started, and the control module 30 retrieves a current treatment plan corresponding to the irradiated body 200 from the treatment plan module 20, and controls irradiation of the neutron beam irradiation device 10 according to the treatment plan. The control module 30 may also receive other data information, such as data of the neutron beam irradiation device 10, data of the irradiated body 200, or the like.

As shown in FIG. 2, in an embodiment, the neutron beam irradiation device 10 includes a neutron generation device 11, a beam shaping body 12, a collimator 13 and a treatment table 14. The neutron generation device 11 includes an accelerator 111 and a target T, the accelerator 111 accelerates charged particles (such as protons, deuterium cores, or the like) to generate a charged particle line such as a proton line, the charged particle line P irradiates to the target T and acts with the target T to generate a neutron line (a neutron beam) N. Preferably, the target T is a metal target. The accelerated charged particles act with the metal target to generate neutrons, and an appropriate nuclear reaction may be selected according to characteristics such as a desired neutron yield and energy, available energies of the accelerated charged particles, a current, physical and chemical properties of the metal target, or the like. Nuclear reactions as commonly discussed include ⁷Li(p, n) ⁷Be and ⁹Be(p, n) ⁹B, both of which are endothermic reactions and have energy thresholds of 1.881 MeV and 2.055 MeV respectively. An ideal neutron source for BNCT is an epithermal neutron at a keV energy level, then theoretically, when protons with energies only slightly higher than the threshold are used to bombard a metallic lithium target, neutrons with relatively low energies may be generated for clinical application without too much retarding treatment. However, proton action sections of lithium (Li) and beryllium (Be) metallic targets with the threshold energy are not high, therefore protons with higher energies are usually selected to initiate a nuclear reaction, to generate a large enough neutron flux. An ideal target should have a high neutron yield, the generated neutron energy distribution is close to an epithermal neutron energy region (it will be described in detail below), there is not too much strong penetrating radiation, and there are characteristics such as safe, cheap, easy to operate, resistant to high temperature, or the like. However, nuclear reactions that meet all requirements may not be found actually, and the target made of metal Li is used in the embodiments of the invention. However, it is well known by those skilled in the art that the target T may also be made of a metal material other than Li and Be, for example, the target T may be formed by tantalum (Ta), tungsten (W) or the like. The target T may be in a shape of a circular plate or another solid shape, or may also use a liquid (liquid metal). The accelerator 111 may be a linear accelerator, a cyclotron, a synchrotron, a synchrocyclotron, and the neutron generation device 11 may also be a nuclear reactor without usage of an accelerator and a target. No matter a neutron source of BNCT comes from the nuclear reactor or charged particles of the accelerator to react with core of the target, a mixed radiation field is generated actually, that is, the beam contains neutrons and photons from low energy to high energy. For BNCT of a tumor at a deep position, except epithermal neutrons, the greater the contents of remaining radiation lines, the greater a proportion of non-selective dose deposition of normal tissues, thus these may reduce unnecessary doses of radiation as much as possible. Furthermore, for normal tissues of the irradiated body, too many kinds of radiation lines should be prevented, which also induces unnecessary dose deposition.

The neutron beam N generated by the neutron generation device 11 sequentially irradiates the irradiated body 200 on the treatment table 14 by passing through the beam shaping body 12 and the collimator 13. The beam shaping body 12 is capable of adjusting a beam quality of the neutron beam N generated by the neutron generation device 11, and the collimator 13 converges the neutron beam N, so that the neutron beam N has high targeting performance during treatment. It may be understood that the invention may not have a collimator, and the beam directly irradiates the irradiated body 200 on the treatment table 14 after coming out of the beam shaping body 12

The beam shaping body 12 further includes a reflector 121, a retarder 122, a thermal neutron absorber 123, a radiation shield 124 and a beam outlet 125. Neutrons generated by the neutron generation device 11 have large energy spectrums, contents of other kinds of neutrons and photons are required to be reduced as much as possible, except that epithermal neutrons meet treatment requirements, to avoid injuries to the operator or the irradiated body. Therefore, neutrons emitted from the neutron generation device 10 are required to pass through the retarder 22 to adjust a fast neutron energy (> 40 KeV) thereof to the epithermal neutron energy region (0.5 eV - 40 keV) and reduce thermal neutrons (< 0.5 eV) as much as possible. The retarder 22 is made of a material having a large cross-section acting with fast neutrons and a small cross-section acting with epithermal neutrons. As a preferred embodiment, and the retarder 122 is made of at least one of D₂O, AlF₃, Fluenal^{™}, CaF₂, Li₂CO₃, MgF₂, or Al₂O₃. The reflector 121 surrounds the retarder 122, and reflects neutrons diffused around by passing through the retarder 122 back to the neutron beam N to improve utilization rate of neutrons, and is made of a material having a strong neutron reflection capability. As a preferred embodiment, the reflector 121 is made of at least one of Pb or Ni, the thermal neutron absorber 123 is arranged at the rear of the retarder 122 and made of a large cross-section acting with epithermal neutrons. As a preferred embodiment, the thermal neutron absorber 123 is made of Li -6, and the thermal neutron absorber 123 absorbs thermal neutrons passing through the retarder 122 to reduce contents of thermal neutrons in the neutron beam N, avoiding excessive dose induced by normal tissues at shallow layers during treatment. It may be understood that the thermal neutron absorber may also be integrated with the retarder, and a material of the retarder contains Li-6. The radiation shield 124 shields neutrons and photons leaked from positions outside of the beam outlet 125, and a material of the radiation shield 124 includes at least one of a photon shielding material or a neutron shielding material. As a preferred embodiment, the material of the radiation shield 124 includes lead (PB) used as the photon shielding material and polyethylene (PE) used as the neutron shielding material. The collimator 13 is arranged at the rear of the beam outlet 125, and an epithermal neutron beam coming out of the collimator 13 irradiates the irradiated body 200, and after passing through the normal tissues at shallow layers, the epithermal neutron beam is slowed down to thermal neutrons to reach a tumor cell M. It may be understood that the beam shaping body 20 may also have other configurations, as long as the epithermal neutron beam required for treatment may be obtained. For ease of description, when the collimator 13 is provided, an outlet of the collimator 13 may also be used as the beam outlet 125 as described below. In this embodiment, a radiation shielding device 15 is further arranged between the irradiated body 200 and the beam outlet 125 to shield radiation coming out of the beam outlet 125 to the normal tissues of the irradiated body, and it may be understood that the radiation shielding device 15 may not be provided.

After a boron (B-10)-containing drug is taken by or injected to the irradiated body 200, the boron-containing drug is selectively aggregated in the tumor cell M, and then two heavily charged particles ⁴He and ⁷Li are generated by using a characteristic of the boron (B-10)-containing drug having a high capture section for a thermal neutron, and through ¹⁰B (n, α) ⁷Li neutron capture and a nuclear fission reaction. The two charged particles have an average energy of about 2.33 MeV, and have characteristics of high linear energy transfer (LET) and short range. LET and range of α particle are 150 keV/µm and 8 µm respectively, LET and range of the heavily charged particle ⁷Li are 175 keV/µm and 5 µm respectively, and the two particles have a total range approximately equivalent to a cell size, so that radiation injury to an organism may be limited to a cell level, and a purpose of locally killing tumor cells may be achieved on premise of not inducing too large injury to normal tissues.

The BNCT system 100 is integrally contained in a building of concrete structure. In particular, the BNCT system 100 further includes an irradiation room 101 and a charged particle beam generation room 102. The irradiated body 200 on the treatment table 14 is subject to irradiation treatment of the neutron beam N in the irradiation room 101. The charged particle beam generation room 102 at least partially contains the accelerator 111, and the beam shaping body 12 is at least partially contained in a partition wall 103 of the irradiation room 101 and the charged particle beam generation room 102. It may be understood that the partition wall 103 may completely separate the irradiation room 101 from the charged particle beam generation room 102; or may be a partial separation between the irradiation room 101 and the charged particle beam generation room 102, so that the irradiation room 101 is in communication with the charged particle beam generation room 102. There may be one or more targets T, the charged particle line P may selectively act with one or more targets T or simultaneously act with multiple targets T to generate one or more treatment neutron beams N. Corresponding to the number of targets T, there may also be one or more beam shaping bodies 12, collimators 13 and treatment tables 14; multiple treatment tables may be arranged in the same irradiation room, or a separate irradiation room may also be provided for each treatment table. The irradiation room 101 and the charged particle beam generation room 102 are spaces formed by surrounding of a concrete wall W (including the partition wall 103), and the concrete structure may shield neutrons and other radiation lines leaked in an operation process of the BNCT system 100.

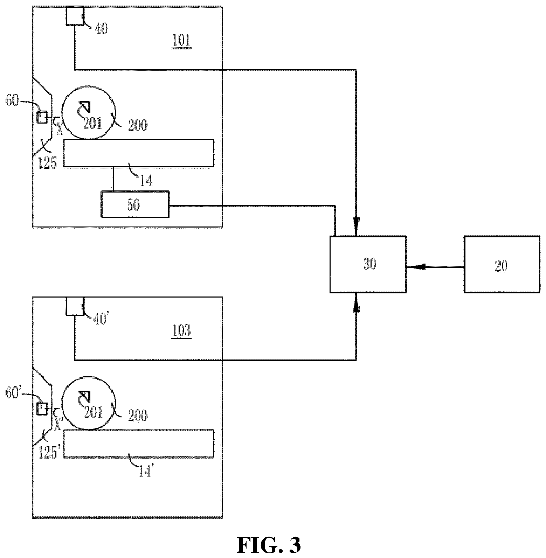

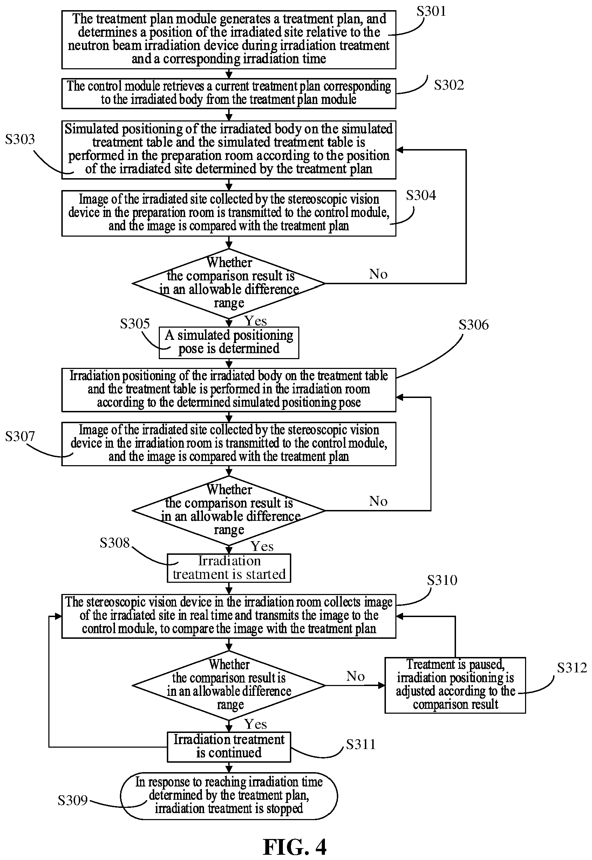

The BNCT system 100 may also include a preparation room 103, a control room (not shown in the figures), and other spaces for auxiliary treatment. Each irradiation room 101 may be provided with a preparation room 103, and the preparation room 103 performs preparation operations such as simulated positioning of the irradiated body 200 before irradiation treatment, boron medicine injection, or the like. The simulated positioning in the preparation room 103 saves operation time of positioning the irradiated body 200 before irradiation treatment in the irradiation room 101, and irradiation treatment of another irradiated body may be performed while performing preparation operations, increasing utilization rate of the device. The operator controls irradiation of the neutron beam irradiation device 10 within the control room. Referring to FIG. 3, the beam outlet 125 of the neutron beam irradiation device 10 is at least partially arranged in the irradiation room 101, the preparation room 103 is provided with a simulated beam outlet 125' which is the same as the beam outlet 125 of the neutron beam irradiation device 10, and a simulated treatment table 14'. The stereoscopic vision device constructs three-dimensional (3D) information of an object by collecting images of the same object at different positions, which may accurately monitor the pose of the irradiated body and ensure that irradiation treatment is performed according to the treatment plan. The irradiation room 101 and the preparation room 103 may be provided with stereoscopic vision devices 40, 40' respectively to collect images of the irradiated body 200 and obtain image data by calculation, such as a coordinate matrix of the irradiated body 200 in a stereoscopic vision device coordinate system, so as to determine position of the irradiated body 200. In an embodiment, two charge coupled device (CCD) cameras are used to form a binocular stereoscopic vision device. It may be understood that the CCD camera may also be replaced by other image acquisition devices, there may be two or more image acquisition devices, which is not specifically limited by the invention. a positional relationship of the beam outlet 125 and the stereoscopic vision device 40 in the irradiation room 101 is the same as that of the simulated beam outlet 125' and the stereoscopic vision device 40' in the preparation room 103, thus the same irradiation coordinate system and stereoscopic vision device coordinate system are defined in the preparation room and the irradiation room. The control module 30 may obtain a coordinate conversion matrix of the irradiation coordinate system and the stereoscopic vision device coordinate system and convert a coordinate matrix of the irradiated body 200 in the stereoscopic vision device coordinate system into a coordinate matrix of the irradiated body in the irradiation coordinate system. That is, the control module 30 converts images of the irradiated body 200 collected by the stereoscopic vision devices 40, 40' into the coordinate matrix of the irradiated body 200 in the irradiation coordinate system. Referring to FIG. 4, an operation process of the radioactive ray irradiation system and a process of positioning the irradiated object according to an embodiment of the invention will be described in detail below.

At S301, the treatment plan module 20 generates a treatment plan, and determines a position of the irradiated site relative to the beam irradiation device 10 during irradiation treatment and a corresponding irradiation time.

At S302, the control module 30 retrieves a current treatment plan corresponding to the irradiated body 200 from the treatment plan module 20.

At S303, simulated positioning of the irradiated body 200 on the simulated treatment table 14' and the simulated treatment table 14' is performed in the preparation room 103 according to the position of the irradiated site determined by the treatment plan.

At S304, image of the irradiated site collected by the stereoscopic vision device 40' in the preparation room 103 is transmitted to the control module 30, and the image is compared with the position of the irradiated site determined by the treatment plan.

When the comparison result is in an allowable difference range, S305 of determining a simulated positioning pose is performed. When the comparison result exceeds the allowable difference range, the process returns to S303, to adjust simulated positioning according to the comparison result.

At S306, irradiation positioning of the irradiated body 200 on the treatment table 14 and the treatment table 14 is performed in the irradiation room 101 according to the determined simulated positioning pose.

At S307, image of the irradiated site collected by the stereoscopic vision device 40 in the irradiation room 101 is transmitted to the control module 30, and the image is compared with the position of the irradiated site determined by the treatment plan.

When the comparison result is in an allowable difference range, S308 of starting irradiation treatment is performed. When the comparison result exceeds the allowable difference range, the process returns to S306, to adjust irradiation positioning according to the comparison result.

At S309, in response to reaching irradiation time determined by the treatment plan, irradiation treatment is stopped.

In an embodiment, the process may further include S310 after S308 of starting irradiation treatment. At S310, the stereoscopic vision device 40 in the irradiation room 101 collects image of the irradiated site in real time and transmits the image to the control module 30, to compare the image with the position of the irradiated site determined by the treatment plan.

When the comparison result is in an allowable difference range, S311 of continuing irradiation treatment is performed, and the process returns to S310 to perform comparison continuously. When the comparison result exceeds the allowable difference range, S312 of pausing treatment and adjusting irradiation positioning according to the comparison result is performed, then the process returns to S310.

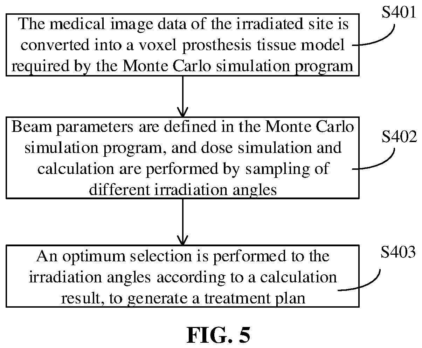

Referring to FIG. 5, S301 further includes S401 to S403.

At S401, the medical image data of the irradiated site is converted into a voxel prosthesis tissue model required by the Monte Carlo simulation program, and data required by an input file of the Monte Carlo simulation program is obtained.

In an embodiment, the medical image data is data of computed tomography (CT), the medical image data of the irradiated site includes a coordinate matrix and a CT value matrix of a medical image voxel model of the irradiated site in a medical image coordinate system, and the treatment plan module converts the CT value matrix into a tissue type information matrix. In BNCT, tissue boron concentration information may also be defined, that is, the voxel prosthesis tissue model required by the Monte Carlo simulation program and provided with the tissue type and the tissue boron concentration information is obtained. It may be understood that other medical image data may be used, as long as the medical image data may be converted into a 3D voxel prosthesis tissue model which may be applied to the radiotherapy system and the method for generating a treatment plan thereof disclosed in the invention. A detailed process of establishing the voxel prosthesis tissue model according to the medical image data may refer to a patent application published on March 8, 2017 with a publication number CN 106474634 A, and entitled "METHOD FOR ESTABLISHING GEOMETRIC MODEL BASED ON MEDICAL IMAGE DATA", which is incorporated here by reference in its entirety.

At S402, beam parameters are defined in the Monte Carlo simulation program (such as Monte Carlo N Particle (MCNP) Transport Code), and dose simulation and calculation are performed by sampling of different irradiation angles.

After the voxel prosthesis tissue model with the tissue type and the tissue boron concentration information is established, collision trajectories and energy distributions of nuclear particles in an internal 3D space when the irradiated body is irradiated by a neutron beam in BNCT may be simulated through the Monte Carlo simulation program. Beam parameters (such as beam energy, intensity, radius, or the like) are defined in the Monte Carlo simulation program, and dose distributions of the 3D voxel prosthesis tissue model at different irradiation angles are simulated and calculated by sampling of different irradiation angles.

At S403, an optimum selection is performed to the irradiation angles according to a calculation result, to determine an optimum irradiation angle of the irradiated site.

After the dose distribution is obtained, an optimum evaluation is performed to different irradiation angles according to a dose index (such as a prescription dose), a dose limit value (such as an average dose and a maximum dose), or the like, to determine the treatment plan, that is, an optimum irradiation angle and a corresponding irradiation time. It may be understood that when the irradiation angles are sampled at S420, or when an optimum evaluation is performed to the irradiation angles at S430, a beam path may also be evaluated, and a method for evaluating a beam angle is not specifically limited by the invention, for example, it may refer to a patent application published on June 16, 2017 with a publication number CN 106853272 A, and entitled "METHOD FOR EVALUATING IRRADIATION ANGLE OF BEAM". The optimal irradiation angle calculated by the treatment plan module determines a position of the irradiated site relative to the neutron beam irradiation device 10. In an embodiment, the treatment plan module 20 may further define, in the Monte Carlo simulation program, an irradiation coordinate system which is the same as the preparation room and the irradiation room. The treatment plan module 20 converts a coordinate matrix of the irradiated site in the medical image coordinate system into a coordinate matrix of the irradiated site in the irradiation coordinate system, and treatment plan data calculated by the treatment plan module includes a coordinate matrix of the voxel prosthesis tissue model of the irradiated site in the irradiation coordinate system at the optimal irradiation angle. It may be understood that conversion of the coordinate matrix of the irradiation coordinate system may not be performed in the Monte Carlo simulation program. Instead, the treatment plan module 20 or the control module 30 calculates a coordinate conversion matrix of the irradiation coordinate system relative to the medical image coordinate system, and performs conversion by the coordinate conversion matrix in subsequent operations so as to perform comparison.

When irradiation treatment is prepared to be started, S302 is performed, the control module 30 retrieves a current treatment plan corresponding to the irradiated body 200 from the treatment plan module 20. The position of the irradiated site may be obtained from the treatment plan, for example, a position of a model of the irradiated site relative to a virtual beam outlet or position parameters related to display may be displayed in a human-computer interface of the control module 30. That is, S303 may be performed, simulated positioning of the irradiated body 200 on the simulated treatment table 14 and the simulated treatment table 14' is performed in the preparation room 103 according to the position of the irradiated site determined by the treatment plan. This process is manually performed by an operator such as a doctor or other people, and is required to be compared and confirmed with the treatment plan. In an embodiment, by S304 of transmitting image of the irradiated site collected by the stereoscopic vision device 40' in the preparation room 103 to the control module 30, the control module 30 converts the image into the irradiation coordinate system, to be compared with the coordinate matrix of the voxel prosthesis tissue model of the irradiated site in the irradiation coordinate system determined by the treatment plan data, to ensure that the comparison result is in an allowable difference range, that is, the simulated positioning pose may be determined (S305). Otherwise, the process returns to S303 to adjust simulated positioning pose, for example, deviation is calculated according to the comparison result, and adjustment is performed according to the calculated deviation; or adjustment is not required. The deviation is taken into account when the irradiated body 200 is positioned in the irradiation room 101. The simulated positioning process takes into account an actual situation of the irradiated body and remains consistent with the irradiation position determined by the treatment plan as much as possible.

In an embodiment, in order to make data collected by the stereoscopic vision device faster and more accurate, a feature pattern 201 is provided on the irradiated site, and a position of the feature pattern 201 in a medical image of the irradiated site is collected. The feature pattern 201 is made of a material which is capable of being developed by a medical image and being recognized by the stereoscopic vision device, a shape of the feature pattern 201 is not limited, and is triangular in the embodiment shown in FIG. 3. It may be understood that this process may be performed before S304 (for example, the feature pattern 201 is provided before the medical image data of the irradiated site is collected in S401, that is, the medical image data of the irradiated site and a position of the feature pattern 201 in the medical image of the irradiated site may be obtained simultaneously), or may be performed in S304. The stereoscopic vision device 40' collects image of the feature pattern 210 and transmits the image to the control module 30, and the control module 30 converts the image into the coordinate matrix of the irradiated site in the irradiation coordinate system through the position of the feature pattern in the medical image of the irradiated site, and compares the coordinate matrix with the coordinate matrix of the voxel prosthesis tissue model of the irradiated site in the irradiation coordinate system determined by the treatment plan data, to ensure that the comparison result is in an allowable difference range, and the simulated positioning pose is determined.

Once simulated positioning is completed, S306 may be performed, irradiation positioning of the irradiated body 200 on the treatment table 14 and the treatment table 14 is performed in the irradiation room 101 according to the determined simulated positioning pose. In an embodiment, a treatment table positioning device 50 is further arranged in the irradiation room 101, and the control module 30 controls movement of the treatment table 14 through the treatment table positioning device 50. It may be understood that a treatment table positioning device may also be arranged in the preparation room 103 to control movement of the simulated treatment table 14', so as to perform simulated positioning of the irradiated body. This process is also manually performed by an operator such as a doctor or other people, and the image of the irradiated site collected by the stereoscopic vision device 40 in the irradiation room 101 is required to be compared and confirmed with the image of the irradiated site corresponding to the simulated positioning pose determined in S305 or the treatment plan, for example, S307 is performed. In an embodiment, the stereoscopic vision device 40 in the irradiation room 101 collects image of the feature pattern, and the control module 30 converts the image into the coordinate matrix of the irradiated site in the irradiation coordinate system through the position of the feature pattern in the medical image of the irradiated site, and compares the coordinate matrix with the coordinate matrix of the voxel prosthesis tissue model of the irradiated site in the irradiation coordinate system determined by the treatment plan data, to ensure that the comparison result is in an allowable difference range, and irradiation treatment may be started (S308). The control module 30 controls the neutron beam generation device 11 to generate a neutron beam, that is, controls the beam irradiation device 10 to start irradiation treatment of the irradiated body 200. Otherwise, the process returns to S306 to adjust the irradiation pose, for example, deviation is calculated according to the comparison result, and adjustment is performed according to the calculated deviation.

In the irradiation treatment process, the stereoscopic vision device 40 in the irradiation room 101 may continuously collect image of the irradiated site in real time and transmit the image to the control module 30, to be compared with a corresponding image of the irradiated site when the irradiation treatment is started in S308 or image of the irradiated site corresponding to the determined simulated positioning pose in S305 or the treatment plan, for example, S310 is performed. In an embodiment, the stereoscopic vision device 40 in the irradiation room 101 collects image of the feature pattern in real time, and the control module 30 converts the image into the coordinate matrix of the irradiated site in the irradiation coordinate system through the position of the feature pattern in the medical image of the irradiated site, and compares the coordinate matrix with the coordinate matrix of the voxel prosthesis tissue model of the irradiated site in the irradiation coordinate system determined by the treatment plan data, to ensure that the comparison result is in an allowable difference range, and irradiation treatment is continued (S311), the process returns to S310 to perform comparison continuously. Otherwise, S312 is performed, to pause treatment, adjust the irradiation pose according to the comparison result and then return to S310. It may be understood that the treatment plan may also be adjusted, and during irradiation treatment, the treatment plan may also be adjusted according to other information received by the control module 30, or other control is performed. Once the control module 30 determines that irradiation time determined by the treatment plan is reached, S309 is performed, the control module controls the beam irradiation device 10 to stop irradiating the irradiated body 200, for example, controls the neutron generation device 11 to stop generating the neutron beam.

In an embodiment, each of the irradiation room 101 and the preparation room 103 is provided with the same laser positioning device 60, 60' having the same positional relationship, and the treatment plan module 20 may simulate a position of a laser generated by the laser positioning device 60, 60' hitting the irradiated site at the irradiation angle determined by the treatment plan. An operator marks on the irradiated body 200 according to the position, and adjusts position and posture of simulated positioning of the irradiated body 200 in the preparation room 103 and position and posture of irradiation positioning of the irradiated body in the irradiation room 101 according to the mark (S303 and S306), so that the position of the laser generated by the laser positioning device 60, 60' hitting the irradiated body 200 is consistent with that of the mark. The laser positioning device is provided, so that manually positioning is more convenient and faster for the operator.

The laser generated by the laser positioning device 60, 60' may also determine a position consistent with central axes X, X' of the beam outlets 125, 125'. As shown in FIG. 3, the position of the laser generated by the laser positioning device 60, 60' hitting the irradiated body 200 represents a position where a central axis of a beam emitted from the beam outlet 125, 125' is incident on the irradiated body 200, and a beam central axis simulated according to the treatment plan is marked on the irradiated body 200 at an incident point of the voxel prosthesis tissue model, so that incident positions of beams determined during the simulated positioning and the irradiation treatment are more accurate.

The treatment plan may also determine sequential irradiation at multiple irradiation angles, for example, please refer to a patent application submitted by the applicant to China National Intellectual Property Administration (CNIPA) on June 11, 2020 with an application number 202010528551.0, and entitled "RADIOTHERAPY SYSTEM AND METHOD FOR GENERATING TREATMENT PLAN THEREOF". After irradiation at a first irradiation angle is completed, simulated positioning at the second irradiation angle is required to be performed again in the preparation room, that is, the above S303 to S312 are repeated.

It may be understood that some simple variations of the calculation method in the above operations, such as simple variations of sequential conversion of different coordinate systems, still fall within the scope of protection of the invention. The concrete wall in the embodiment is a boron-containing barite concrete wall with a thickness of 1m or more and a density of 3g/c.c. The boron-containing concrete has better neutron absorption performance, and in addition to enhancing a radiation shielding effect of the concrete, neutron exposure of a metal material in the concrete may also be reduced. It may be understood that the concrete wall may also have another thickness or density, or may be replaced by other materials, and the concrete wall may have different thicknesses, densities or materials at different portions. It may be understood that the invention may also be applied to other types of neutron irradiation systems, and may also be applied to other radioactive ray irradiation systems, such as proton therapy systems, heavy ion therapy systems, or the like. At this time, the neutron generation device may be replaced by other radioactive ray generation devices, the material of the concrete may be replaced as required, and the treatment table may also be a carrying table of other irradiated bodies.

It may be understood that the preparation room and the stereoscopic vision device in the preparation room may not be provided, irradiation positioning is directly performed in the irradiation room according to the position of the irradiated site determined by the treatment plan before irradiation treatment is started, and the irradiation positioning is compared with the treatment plan. Specifically, the radioactive ray irradiation system includes a beam irradiation device, a treatment plan module, a control module, a preparation room and an irradiation room. The beam irradiation device generates a treatment beam and irradiates the treatment beam to an irradiated body to form an irradiated site. The treatment plan module performs dose simulation and calculation according to parameters of the treatment beam and medical image data of the irradiated site and generates a treatment plan which determines a position of the irradiated site relative to the beam irradiation device during irradiation treatment. The control module controls irradiation of the beam irradiation device according to the treatment plan. Irradiation positioning of the irradiated body is performed in the irradiation room according to the position of the irradiated site determined by the treatment plan, and a stereoscopic vision device is arranged in the irradiation room to collect image of the irradiated site, and the control module compares the image with the position of the irradiated site determined by the treatment plan, to ensure that the comparison result is in an allowable difference range and control the beam irradiation device to start irradiation treatment of the irradiated body. The control method for the radioactive ray irradiation system includes the following operations. The treatment plan module performs dose simulation and calculation according to parameters of the treatment beam generated by the beam irradiation device and medical image data of the irradiated site, and generates a treatment plan which determines a position of the irradiated site relative to the beam irradiation device during irradiation treatment and a corresponding irradiation time. The control module retrieves a current treatment plan corresponding to the irradiated body from the treatment plan module. Irradiation positioning of the irradiated body is performed in the irradiation room according to the position of the irradiated site determined by the treatment plan. The stereoscopic vision device collects image of the irradiated site, and the control module compares the image with the position of the irradiated site determined by the treatment plan, to ensure that the comparison result is in an allowable difference range and control the beam irradiation device to start irradiation treatment of the irradiated body. In response to reaching irradiation time determined by the treatment plan, the control module controls the beam irradiation device to stop irradiation of the irradiated body.

While the illustrative specific implementations of the invention have been described as above, so that those skilled in the art understand the invention, it should be apparent that the invention is not limited to the scope of the specific implementations, various changes are apparent for those of ordinary skill in the art and fall within the scope of protection of the invention, as long as these changes fall within the spirit and scope of the invention as defined and determined by the appended claims.

## Claims

1. A radioactive ray irradiation system, **characterized in that** the radioactive ray irradiation system comprises:
a beam irradiation device generating a treatment beam and irradiating the treatment beam to an irradiated body to form an irradiated site;
a treatment plan module performing dose simulation and calculation according to parameters of the treatment beam and medical image data of the irradiated site and generating a treatment plan which determines a position of the irradiated site relative to the beam irradiation device during irradiation treatment;
a control module controlling irradiation of the beam irradiation device according to the treatment plan;
a preparation room in which simulated positioning of the irradiated body is performed according to the position of the irradiated site determined by the treatment plan, and in which a first stereoscopic vision device is arranged to collect a first image of the irradiated site, and the control module comparing the first image with the position of the irradiated site determined by the treatment plan, to ensure that the comparison result is in an allowable difference range and determine a simulated positioning pose of the irradiated body; and
an irradiation room in which irradiation position of the irradiated body is performed according to the determined simulated positioning pose, and in which a second stereoscopic vision device is arranged to collect a second image of the irradiated site, the control module comparing the second image with the first image of the irradiated site corresponding to the determined simulated positioning pose or the position of the irradiated site determined by the treatment plan, to ensure that the comparison result is in an allowable difference range, and control the beam irradiation device to start irradiation treatment of the irradiated body.

2. The radioactive ray irradiation system of claim 1, wherein the beam irradiation device comprises a beam outlet at least partially arranged in the irradiation room, the preparation room is provided with a simulated beam outlet which is the same as the beam outlet, a positional relationship of the simulated beam outlet and the first stereoscopic vision device is the same as that of the beam outlet and the second stereoscopic vision device, the preparation room and the irradiation room define the same irradiation coordinate system therein, and the control module is capable of converting each of the first image and the second image into a coordinate matrix of the irradiated site in the irradiation coordinate system.

3. The radioactive ray irradiation system of claim 2, wherein the treatment plan module performs dose simulation and calculation by using a Monte Carlo simulation program, the treatment plan module converts the medical image data of the irradiated site into a voxel prosthesis tissue model required by the Monte Carlo simulation program, the medical image data of the irradiated site comprises a coordinate matrix of the irradiated site in a medical image coordinate system, and the treatment plan module or the control module is capable of acquiring a coordinate conversion matrix of the medical image coordinate system and the irradiation coordinate system.

4. The radioactive ray irradiation system of claim 2, wherein the irradiated site is provided with a feature pattern made of a material which is capable of being developed by a medical image and being recognized by the first stereoscopic vision device and the second stereoscopic vision device, each of the first stereoscopic vision device and the second stereoscopic vision device collects an image of the irradiated site by collecting an image of the feature pattern, and the control module converts the image of the feature pattern into the coordinate matrix of the irradiated site in the irradiation coordinate system according to a position of the feature pattern in a medical image of the irradiated site.

5. The radioactive ray irradiation system of claim 2, wherein each of the preparation room and the irradiation room is provided with the same laser positioning device having the same positional relationship, and the treatment plan module is capable of simulating a position of a laser generated by the laser positioning device hitting the irradiated site.

6. The radioactive ray irradiation system of claim 5, wherein the laser generated by the laser positioning device determines a position consistent with central axes of the beam outlet and the simulated beam outlet.

7. The radioactive ray irradiation system of claim 1, further comprising a treatment table and a treatment table positioning device arranged in the irradiation room, the irradiated body is subject to irradiation treatment on the treatment table, and the control module controls movement of the treatment table through the treatment table positioning device.

8. The radioactive ray irradiation system of claim 1, wherein the second stereoscopic vision device collects a third image of the irradiated site in real time during irradiation treatment, and the control module compares the third image with a corresponding second image of the irradiated site when the irradiation treatment is started, or the first image of the irradiated site corresponding to the determined simulated positioning pose or the position of the irradiated site determined by the treatment plan, to ensure that the comparison result is in an allowable difference range, and control the beam irradiation device to continuously perform the irradiation treatment of the irradiated body.

9. The radioactive ray irradiation system of claim 1, wherein the radioactive ray irradiation system is a neutron capture therapy system, the beam irradiation device comprises: a neutron generation device comprising an accelerator and a target, the accelerator accelerating charged particles to generate a charged particle line which acts with the target to generate a neutron line; a beam shaping body capable of adjusting the neutron line generated by the neutron generation device to a preset beam quality; and a treatment table on which the irradiated body is irradiated by the neutron line generated by the neutron generation device through the beam shaping body.

10. A control method for a radioactive ray irradiation system, **characterized in that** the radioactive ray irradiation system comprises a beam irradiation device generating a treatment beam and irradiating the treatment beam to an irradiated body to form an irradiated site, a treatment plan module, a control module, a preparation room and an irradiation room in which a first stereoscopic vision device and a second stereoscopic vision device are arranged respectively, the control method comprising:
performing, by the treatment plan module, dose simulation and calculation according to parameters of the treatment beam generated by the beam irradiation device and medical image data of the irradiated site, and generating, by the treatment plan module, a treatment plan which determines a position of the irradiated site relative to the beam irradiation device during irradiation treatment and a corresponding irradiation time;
retrieving, by the control module, a current treatment plan corresponding to the irradiated body from the treatment plan module;
performing simulated positioning of the irradiated body in the preparation room according to the position of the irradiated site determined by the treatment plan;
collecting, by the first stereoscopic vision device, a first image of the irradiated site, and comparing, by the control module, the first image with the position of the irradiated site determined by the treatment plan, to ensure that the comparison result is in an allowable difference range and determine a simulated positioning pose of the irradiated body;
performing irradiation positioning of the irradiated body in the irradiation room according to the determined simulated positioning pose;
collecting, by the second stereoscopic vision device, a second image of the irradiated site, and comparing, by the control module, the second image with the first image of the irradiated site corresponding to the determined simulated positioning pose or the position of the irradiated site determined by the treatment plan, to ensure that the comparison result is in an allowable difference range, and control the beam irradiation device to start irradiation treatment of the irradiated body; and
in response to reaching irradiation time determined by the treatment plan, controlling, by the control module, the beam irradiation device to stop irradiation of the irradiated body.

11. The control method of claim 10, further comprising: after the beam irradiation device starts the irradiation treatment of the irradiated body, collecting, by the second stereoscopic vision device, a third image of the irradiated site in real time during the irradiation treatment, and comparing, by the control module, the third image with a corresponding second image of the irradiated site when the irradiation treatment is started, or the first image of the irradiated site corresponding to the determined simulated positioning pose or the position of the irradiated site determined by the treatment plan, to ensure that the comparison result is in an allowable difference range, and control the beam irradiation device to continuously perform the irradiation treatment of the irradiated body.

12. The control method of claim 10, further comprising: defining the same irradiation coordinate system in the preparation room and the irradiation room; converting, by the treatment plan module, the medical image data of the irradiated site into a voxel prosthesis tissue model; and converting, by the treatment plan module, the position of the irradiated site determined by the treatment plan into a coordinate matrix of the voxel prosthesis tissue model of the irradiated site in the irradiation coordinate system.

13. The control method of claim 12, further comprising: converting, by the control module, the first image and the second image into the irradiation coordinate system, for comparison.

14. The control method of claim 12, further comprising: providing a feature pattern on the irradiated site, and collecting a position of the feature pattern in a medical image of the irradiated site.

15. The control method of claim 14, further comprising: collecting, by the first stereoscopic vision device and the second stereoscopic vision device, images of the feature pattern and transmitting, by the first stereoscopic vision device and the second stereoscopic vision device, the images to the control module; and converting, by the control module, the images into coordinate matrices of the irradiated site in the irradiation coordinate system through the position of the feature pattern in the medical image of the irradiated site, for comparison.
